# EUROPÄISCHE PATENTSCHRIFT

(11) **EP 3 031 416 B1**
(45) Veröffentlichungstag und Bekanntmachung des Hinweises auf die Patenterteilung: **10.04.2019**
(21) Anmeldenummer: 15187873.3
(22) Anmeldetag: 01.10.2015
(51) Int. Cl.: A61B 17/70

(54) **OSTEOSYNTHESEVORRICHTUNG**
OSTEOSYNTHESIS DEVICE
DISPOSITIF D'OSTEOSYNTHESE

(30) Priorität: 09.12.2014 DE 102014225327
(43) Veröffentlichungstag der Anmeldung: 15.06.2016
(73) Patentinhaber: Silony Medical International AG, 8500 Frauenfeld (CH)
(72) Erfinder: Halm, Henry, 48151 Münster (DE)
(74) Vertreter: DREISS Patentanwälte PartG mbB

(56) Entgegenhaltungen:
- EP-B1- 2 292 169
- WO-A1-2004/041100
- US-A1- 2014 031 873

## Beschreibung

Die Erfindung betrifft eine Sachgesamtheit aus einer Osteosynthesevorrichtung und einem Handhabungsinstrument. Sie geht aus von einer Osteosynthesevorrichtung mit einem einen Schaft und einen Kopf aufweisenden Knochenanker, insbesondere einer Knochenschraube, und mit einem in einer Seitenansicht U-förmigen und eine Aufnahmeöffnung für ein Korrekturelement, insbesondere einen Korrekturstab, aufweisenden Gabelkopf mit zwei Schenkeln, wobei der Kopf des Knochenankers und der Gabelkopf entweder miteinander starr, insbesondere einstückig ausgebildet sind oder der Kopf des Knochenankers an einem distalen Ende des Gabelkopfs verschwenkbar gelagert ist und in diesem Fall der Gabelkopf in einer vom Chirurgen intendierten Schwenkstellung bezüglich des Kopfs des im Knochen festgelegten oder festlegbaren Knochenankers fixierbar ist, wobei der Gabelkopf eine Längsrichtung und eine hierzu radiale Richtung aufweist sowie ein distales Ende benachbart zu dem Knochenanker und ein hiervon in Längsrichtung abgewandtes proximales Ende aufweist, so dass auch eine distale Richtung und eine proximale Richtung definiert ist, wobei sich die Schenkel ausgehend von einem distalen Bereich des Gabelkopfs in proximaler Richtung erstrecken und zwischen sich die Aufnahmeöffnung für das Korrekturelement begrenzen, wobei die Schenkel einen radial äußeren Umfangsbereich aufweisen, der bogenförmig, insbesondere kreisbogenförmig gekrümmt ausgebildet ist und in dem zum Ergreifen des Gabelkopfs mittels eines Handhabungsinstruments jeweils wenigstens eine Haltenut ausgebildet ist, die sich in einer Umfangsrichtung des Gabelkopfs und in einer Ebene orthogonal zur Längsrichtung des Gabelkopfs erstreckt.

Osteosynthesevorrichtungen betreffen insbesondere das Gebiet der Wirbelsäulenchirurgie, um benachbarte Wirbelkörper auszurichten und in einer gewünschten Position relativ zueinander zu fixieren. Hierbei wird in benachbarte Wirbelkörper jeweils ein Knochenanker eingebracht, und zwar typischerweise als Knochenschraube eingeschraubt, und es werden dann diese benachbarten Knochenanker über ein Korrekturelement, typischerweise über einen Korrekturstab, verbunden, der in dem jeweiligen Knochenanker in einer bestimmten und vom Chirurgen erwünschten Position geklemmt wird. Auf diese Weise werden benachbarte Wirbelkörper ausgerichtet und in einer gewünschten Position relativ zueinander fixiert. Zum Ergreifen und Handhaben des Knochenankers beim Einbringen oder Ausrichten während des chirurgischen Eingriffs kann ein Handhabungsinstrument verwendet werden, welches durch Eingriff in die Haltenut an dem Knochenanker lösbar, jedoch vorzugsweise verliersicher mit diesem verbindbar ist.

Derartige Osteosynthesevorrichtungen sind bekannt, beispielsweise aus WO 2004/041100 A1, WO 03/028566 A1, FR 2796545 A1, EP 1 190 678 A2, EP 2 292 169 B1, DE 296 06 468 U1. Bei diesen vorbekannten Osteosynthesevorrichtungen ist die betreffende Haltenut entweder in der radialen Richtung nicht hinterschnitten, oder es ist eine zur Längsrichtung der Osteosynthesevorrichtung schräg geneigte Nutflanke vorgesehen, die nicht unter allen Betriebsbedingungen eine verliersichere, jedoch lösbare Halteverbindung zwischen Osteosynthesevorrichtung und Handhabungsinstrument gewährleistet. US 2014/0031873 A1 offenbart eine Osteosynthesevorrichtung mit einer rechteckförmig hinterschnittenen Haltenut, wobei das entsprechend angepasste Handhabungsinstrument einen hierzu komplementären Vorsprung aufweist und in eine Hintergriffsstellung um die Längsrichtung verdrehbar ist.

Demgemäß liegt der vorliegenden Erfindung die Aufgabe zugrunde, eine noch zuverlässigere lösbare Halteverbindung bereitzustellen.

Diese Aufgabe wird durch eine Sachgesamtheit mit den Merkmalen des Anspruchs 1 gelöst. Mit der vorliegenden Erfindung wird eine noch sicherere wenn auch lösbare Halteverbindung zwischen der Osteosynthesevorrichtung und dem Handhabungsinstrument realisiert. Es wurde nämlich festgestellt, dass bei manchen Handhabungsbedingungen nicht nur Kräfte in distaler Längsrichtung in Richtung des oberen Raumwinkels bis zur "Horizontalen", also bis zur radialen Richtung, eingeleitet werden, sondern es kann mitunter vorkommen, dass auch eine kurzzeitige Kraftbeaufschlagung in leicht proximaler Richtung, also unterhalb der "Horizontalen" oder radialen Richtung, auftreten. In diesen Fällen haben sich die vorbekannten Osteosynthesevorrichtungen und deren Ausbildung der Haltenut nicht stets als optimal erwiesen. Dadurch, dass bei der neuen erfindungsgemäßen Gestaltung der zweite Flankenabschnitt in proximaler Richtung erstreckt ist und konzentrisch zur Längsrichtung des Gabelkopfs verläuft, kann auch unter diesen Handhabungsbedingungen eine verliersichere Anordnung des Instruments realisiert werden.

Bevorzugte Weiterbildungen sind in den abhängigen Ansprüchen angegeben und beansprucht.

Weitere Merkmale, Einzelheiten und Vorteile der Erfindung ergeben sich aus den abhängigen Ansprüchen, für die in beliebiger Kombination Schutz in Anspruch genommen wird, sowie aus der zeichnerischen Darstellung eines bevorzugten Ausführungsbeispiels der erfindungsgemäßen Osteosynthesevorrichtung. In der Zeichnung zeigt:
- Figur 1a: eine die Längsrichtung einschließende Schnittansicht eines Gabelkopfs der erfindungsgemäßen Osteosynthesevorrichtung mit Schnittebene A-A in Figur 1b;
- Figur 1b: eine Draufsicht auf den Gabelkopf nach Figur 1a;
- Figur 1c: eine Einzelheit Z aus Figur 1a, welche die Gestaltung einer Haltenut verdeutlicht;
- Figur 2a: eine Längsschnittansicht einer erfindungsgemäßen Osteosynthesevorrichtung mit Gabelkopf und Knochenanker in Form einer Knochenschraube;
- Figur 2b: eine Einzelheit Z aus Figur 2a, welche die Gestaltung der Haltenut verdeutlicht;
- Figur 3a: eine Längsschnittansicht einer erfindungsgemäßen Osteosynthesevorrichtung nach Figuren 1 und 2 und eines daran lösbar gehaltenen Handhabungsinstruments;
- Figur 3b: eine Einzelheit Z aus Figur 3a, aus der der Halteeingriff des Instruments an der Haltenut des Gabelkopfs ersichtlich ist.

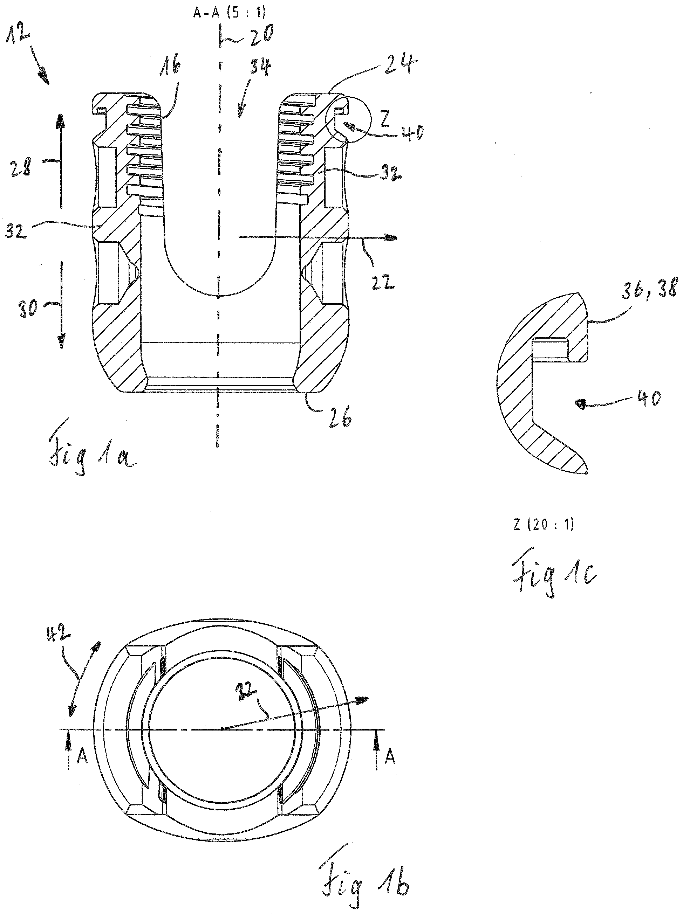

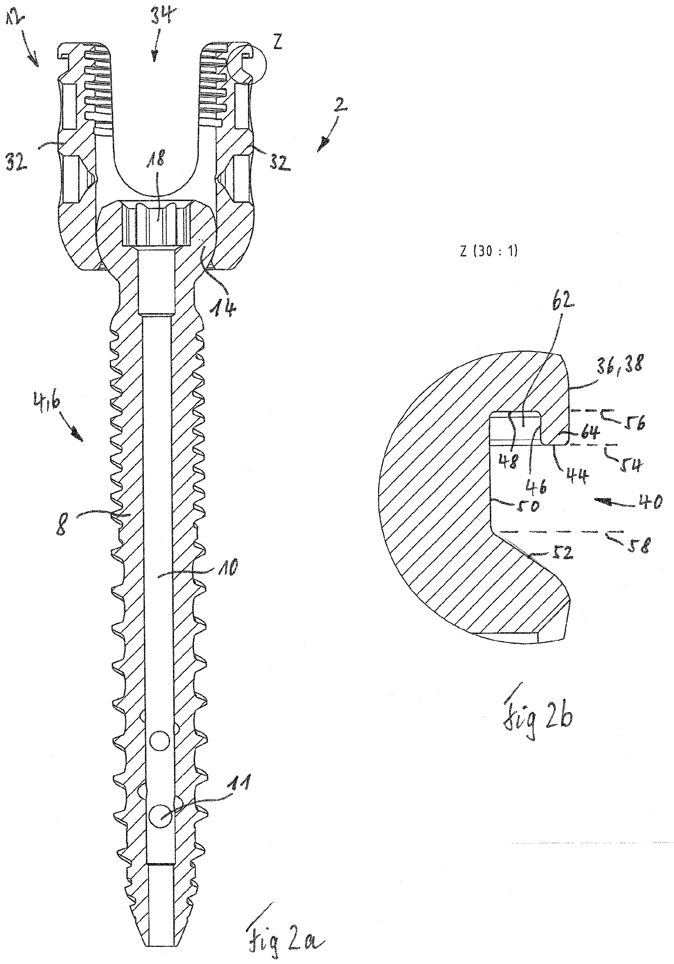

Die Figuren 1a-c) und 2a, b) zeigen eine insgesamt mit dem Bezugszeichen 2 bezeichnete erfindungsgemäße Osteosynthesevorrichtung. Sie umfasst einen Knochenanker 4, der vorliegend beispielhaft und bevorzugterweise als Knochenschraube 6 ausgebildet ist und demgemäß einen Gewindeschaft 8 aufweist. Der Knochenanker 4 bzw. dessen Gewindeschaft 8 ist kannüliert ausgebildet, das heißt er weist eine durchgehende Längsöffnung 10 sowie Queröffnungen 11 zum Ausbringen von Knochenzement zwischen Gewindeschaft 8 und Knochen des Patienten auf. Die Längsöffnung 10 kann aber auch mit einem zu einer Bohrung im Knochen führenden Führungsdraht zusammenwirken, um den Knochenanker 4 zu der intendierten Öffnung zu dirigieren. Die Osteosynthesevorrichtung 2 umfasst des Weiteren einen noch näher zu beschreibenden Gabelkopf 12. Der Gabelkopf 12 dient zur Aufnahme des Knochenankers 4, wobei der Knochenanker 4 hierfür an seinem proximalen Ende einen Kopf 14 aufweist, der nach Art eines Kalottenlagers in einem distalen Lagerbereich des Gabelkopfs 12 in verschiedenen und vom Chirurgen einzustellenden Positionen relativ zum Gabelkopf 12 festgelegt werden kann. Hierfür wird vom proximalen Ende des Gabelkopfs 12 ausgehend, über nicht dargestellte Klemmelemente, beispielsweise eine Madenschraube und gegebenenfalls weitere Druckstücke eine Klemmkraft auf den Kopf des Knochenankers 4 ausgeübt. Zur Aufnahme eines solchen Klemmelements weist der Gabelkopf im beispielhaft dargestellten Fall in einem proximalen Bereich ein Innengewinde 16 auf. Vor dem Festziehen des Klemmelements wird zwischen dem Knochenanker 4 und das Klemmelement ein an sich bekanntes aber nicht dargestelltes Korrekturelement, insbesondere ein Korrekturstab, eingelegt, so dass durch Festziehen des Klemmelements die gesamte Anordnung fixierbar ist, was typischerweise erst geschieht, wenn der Knochenanker 4 in seiner bestimmungsgemäßen Position in den Knochen eingebracht ist. Dies geschieht wiederum typischerweise über ein nicht dargestelltes Werkzeug, welches mit einer Werkzeugansetzstelle 18 am Kopf 14 des Knochenankers 4 zusammenwirkt. Danach werden der Gabelkopf 12 und der eingelegte Korrekturstab relativ zu dem Knochenanker 4 ausgerichtet und in der vom Chirurgen intendierten Position durch Festziehen des Klemmelements fixiert.

In Figuren 2a, b) ist eine Längsrichtung 20 sowie eine hierzu radiale Richtung 22 eingezeichnet. Der Gabelkopf 12 weist ferner ein proximales Ende 24 und ein distales Ende 26 auf, so dass, ausgehend von dem distalen Ende 24 eine proximale Richtung 28 und ausgehend von dem proximalen Ende 24 eine distale Richtung 30 unterscheidbar sind.

Der Gabelkopf 12 ist in der Ansicht der Figuren 1a und 2a, also in der Seitenansicht U-förmig gestaltet. Er weist zwei Schenkel 32 auf, die sich ausgehend vom distalen Ende 26 des Gabelkopfs 12 in proximaler Richtung 28 erstrecken und frei enden. Diese Schenkel 32 bilden eine topfförmige Wandung, wobei die Schenkel 32 zwischen sich eine Aufnahmeöffnung 34 zur Aufnahme des schon erwähnten, jedoch nicht dargestellten Korrekturstabs und des Klemmelements definieren.

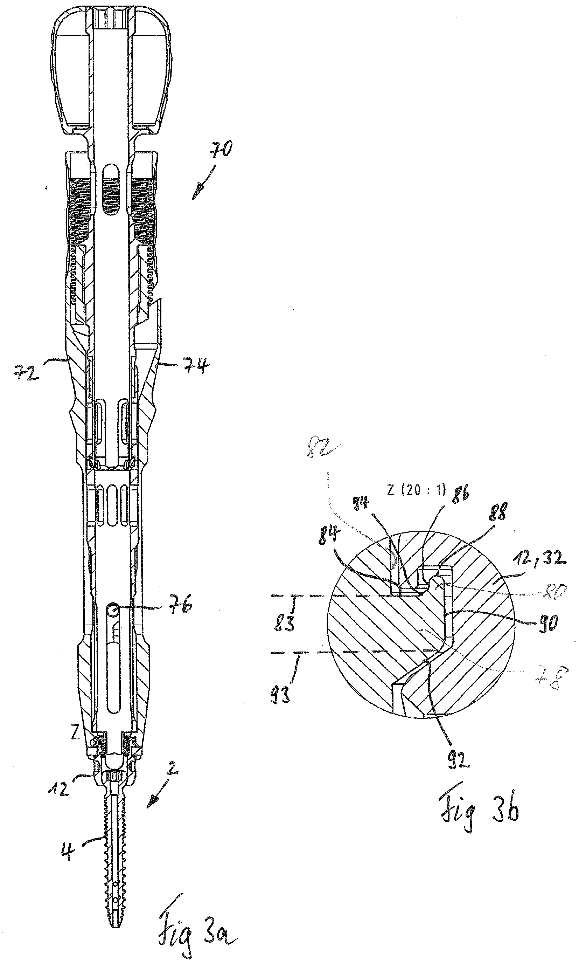

Radial außen bilden die Schenkel 32 jeweils einen Umfangsbereich 36 des Gabelkopfs 12. Ausgehend von einer Oberfläche 38 dieses Umfangsbereichs 36 ist eine insgesamt mit dem Bezugszeichen 40 bezeichnete Haltenut ausgebildet, die sich in einer Umfangsrichtung 42 des Gabelkopfs 12 und in einer Ebene orthogonal zur Längsrichtung 20 des Gabelkopfs 12 erstreckt. Die Haltenut dient zum lösbaren Fixieren eines in Figur 3a dargestellten Handhabungsinstruments zum Halten, Einbringen und Manipulieren der Osteosynthesevorrichtung während des chirurgischen Eingriffs, sie wird nachfolgend im Einzelnen anhand Figur 2b erläutert werden.

Figur 2b zeigt als Einzelheit Z von Figur 2a die Haltenut 40, wobei die Zeichnungsebene in einer die Längsrichtung 20 des Gabelkopfs 12 einschließenden Schnittebene gemäß Schnittebene von Figur 2a dargestellt ist. Ausgehend von der Oberfläche 38 des Umfangsbereichs 36 im Bereich des proximalen Endes 24 des Gabelkopfs 12 wird die Haltenut 40 begrenzt von einem ersten bis fünften Flankenabschnitt 44, 46, 48, 50 bzw. 52. Der erste Flankenabschnitt 44 erstreckt sich nach radial innen und verläuft in einer Ebene 54 orthogonal zur Längsrichtung 20 des Gabelkopfs 12. Daran anschließend erstreckt sich der zweite Flankenabschnitt 46 in proximaler Richtung 28 und verläuft konzentrisch zur Längsrichtung 20 des Gabelkopfs 12. Daran anschließend erstreckt sich der dritte Flankenabschnitt 48 nach radial innen und verläuft in einer Ebene 56 orthogonal zur Längsrichtung 20 des Gabelkopfs 12. Daran anschließend erstreckt sich der vierte Flankenabschnitt 50 in distaler Richtung 30 und verläuft konzentrisch zur Längsrichtung 20 des Gabelkopfs 12. Er erstreckt sich dabei in distaler Richtung 30 bis unter den ersten Flankenabschnitt 44. Anschließend an das distale Ende 30 des vierten Flankenabschnitts 50 erstreckt sich der fünfte Flankenabschnitt 52 mit einer Komponente nach radial außen und verläuft im beispielhaft dargestellten Fall in einem Winkel zu einer Ebene 58 orthogonal zur Längsrichtung 20 des Gabelkopfs 12, also distal geneigt zur Horizontalen. Es wäre auch denkbar, dass der fünfte Flankenabschnitt ganz in der Ebene 58 in radialer Richtung 22 erstreckt ist.

Die Haltenut 40 bildet daher einen in radialer Richtung 22 hintergreifbaren Bereich 62. Dieser Bereich 62 ist radial außen begrenzt von dem zweiten Flankenabschnitt 46. Man erkennt einen in der Schnittansicht der Figur 2b ungefähr rechteckförmigen Bund 64 zwischen zweitem Flankenabschnitt 46 und Oberfläche 38 des Umfangsbereichs 36, der in distaler Richtung 30 über den dritten Flankenabschnitt 48 in Längsrichtung 20 vorsteht.

Figur 3a zeigt ein insgesamt mit dem Bezugszeichen 70 bezeichnetes Handhabungsinstrument in lösbarer Halteverbindung mit der vorausgehend beschriebenen Osteosynthesevorrichtung 2. Anhand der Einzelheit Z gemäß Figur 3b ist diese lösbare Halteverbindung ersichtlich. Das Handhabungsinstrument 70 umfasst zwei zueinander zangenartig beschränkt auslenkbare Haltebacken 72, 74, deren Drehpunkt mit Bezugszeichen 76 dargestellt ist. Die Haltebacken 72, 74 weisen jeweils einen nach radial innen vorstehenden Vorsprung 78 mit dem sie in die jeweilige Haltenut 40 eingreifen und dort in verliersicherem Hintergriff stabil gehalten werden können. Hierfür weist der jeweilige Vorsprung 78 einen in proximaler Richtung 28 erstreckten Ansatz 80 auf, welcher in den hintergreifbaren Bereich 62 der Haltenut 40 eingreifen kann und so einen radialen Hintergriff mit dem Bund 64 des Gabelkopfs 12 ausbildet.

Der Vorsprung 78 wird durch folgende Flächenabschnitte begrenzt: Er weist einen ersten nach radial innen erstreckten und in einer Ebene 83 orthogonal zur Längsrichtung 20 des Gabelkopfs 12 bzw. des Handhabungsinstruments 70 verlaufenden Flächenabschnitt 84 auf.

Des Weiteren ist der Vorsprung begrenzt durch einen zweiten Flankenabschnitt 86, der in proximaler Richtung 28 erstreckt ist und konzentrisch zur Längsrichtung 20 verläuft. Mit diesem zweiten Flächenabschnitt 86 ist der Ansatz 80 des Vorsprungs 78 von radial innen an den zweiten Flankenabschnitt 46 des Gabelkopfs 12 flächenhaft anlegbar. Weiter ist ein dritter Flächenabschnitt 88 vorgesehen, welcher den Ansatz 80 des Vorsprungs 78 nach oben hin, also proximal begrenzt und im beispielhaft dargestellten Fall verrundet ausgebildet ist. Ein vierter Flächenabschnitt 90 ist, ausgehend von dem dritten Flächenabschnitt 88 in distaler Richtung 30 erstreckt und verläuft konzentrisch zur Längsrichtung 20. Er erstreckt sich in distaler Richtung 30 bis unter den ersten Flächenabschnitt 84. Sodann wird der Vorsprung 78 distal durch einen fünften Flächenabschnitt 92 begrenzt, der im beispielhaft dargestellten Fall in einem Winkel distal geneigt zu einer Ebene 93orthogonal zur Längsrichtung 20 verläuft. Im beispielhaft dargestellten Fall ist der fünfte Flächenabschnitt 92 des Vorsprungs 78 parallel zu dem fünften Flankenabschnitt 52 der Haltenut 40 ausgebildet.

Man erkennt in Figur 3b des Weiteren zwischen dem ersten und dem zweiten Flächenabschnitt 84 bzw. 86 einen weiteren beispielhaft linear verlaufenden Flächenabschnitt 94, der aber auch bogenförmig gekrümmt verlaufen könnte. Dieser weitere Flächenabschnitt 94 ist nicht zwingend erforderlich, jedoch insoweit vorteilhaft, als er eine Art Aufgleitrampe für den Bund 64 des Gabelkopfs 12 bildet.

Figur 3a zeigt das Handhabungsinstrument 70 im lösbaren Eingriff mit der Haltenut 40 des Gabelkopfs 12. In dieser lösbaren Verbindung kann die Osteosynthesevorrichtung 2 mit Hilfe des Handhabungsinstruments 70 manipuliert werden. Insbesondere kann die Osteosynthesevorrichtung 2 so zu einer zuvor ausgebildeten Öffnung im Knochen gebracht werden. Es ist dann möglich, durch das Handhabungsinstrument 70 hindurch mittels eines nicht dargestellten schraubenzieherartigen Instruments Zugriff zu dem Knochenanker 4 zu nehmen und diesen in den Knochen einzuschrauben. Danach kann wiederum durch das Handhabungsinstrument 70 hindurch das Klemmmittel eingebracht und in das Innengewinde 16 eingeschraubt werden, wodurch dann in der entsprechenden vom Chirurgen gewünschten Ausrichtung die Osteosynthesevorrichtung 2 mit einem nicht dargestellten Korrekturelement dauerhaft gegenüber dem Knochen des Patienten fixiert und ausgerichtet werden kann. Währenddessen kann auch über das Handhabungsinstrument 70 eine Kraft zum erwünschten Ausrichten des Knochens oder der Wirbel relativ zu benachbarten Osteosynthesevorrichtungen realisiert werden. Hierbei werden mitunter große Kräfte übertragen. Die erfindungsgemäße Osteosynthesevorrichtung gestattet hierbei eine verliersichere Kopplung zwischen Handhabungsinstrument 70 und Gabelkopf 12, so dass ein Lösen in radialer Richtung sicher verhindert wird, indem eine wohl definierte Anschlagfläche zwischen dem Bund 64 und dem Vorsprung 78 bzw. dessen Ansatz 80 realisiert wird, und zwar auch wenn zeitweilig Kräfte in Richtung unterhalb einer Ebene orthogonal zur Längsrichtung eingeleitet werden.

## Patentansprüche

1. Sachgesamtheit aus einer Osteosynthesevorrichtung (2) und einem Handhabungsinstrument (70) zum Ergreifen eines Gabelkopfs (12) der Osteosynthesevorrichtung (2) durch beidseitigen Eingriff in eine jeweilige Haltenut (40) des Gabelkopfs (12),
wobei die Osteosynthesevorrichtung (2) ausgebildet ist mit einem einen Schaft (8) und einen Kopf (14) aufweisenden Knochenanker (4), insbesondere einer Knochenschraube (6), und mit einem in einer Seitenansicht U-förmigen und eine Aufnahmeöffnung (34) für ein Korrekturelement, insbesondere einen Korrekturstab, aufweisenden Gabelkopf (12) mit zwei Schenkeln (32), wobei der Kopf (14) des Knochenankers (4) und der Gabelkopf (12) entweder miteinander starr, insbesondere einstückig ausgebildet sind oder der Kopf (14) des Knochenankers (4) an einem distalen Ende (26) des Gabelkopfs (12) verschwenkbar gelagert ist und in diesem Fall der Gabelkopf (12) in einer vom Chirurgen intendierten Schwenkstellung bezüglich des Kopfs (14) des im Knochen festgelegten oder festlegbaren Knochenankers (4)fixierbar ist, wobei der Gabelkopf (12) eine Längsrichtung (20) und eine hierzu radiale Richtung (22) aufweist sowie ein distales Ende (26) benachbart zu dem Knochenanker (4) und ein hiervon in Längsrichtung (20) abgewandtes proximales Ende (24) aufweist, so dass auch eine distale Richtung (30) und eine proximale Richtung (28) definiert sind, wobei sich die Schenkel (32) ausgehend von einem distalen Bereich des Gabelkopfs (12) in proximaler Richtung (28) erstrecken und zwischen sich die Aufnahmeöffnung (34) für das Korrekturelement begrenzen, wobei die Schenkel (32) einen radial äußeren Umfangsbereich (36) aufweisen, der bogenförmig, insbesondere kreisbogenförmig gekrümmt ausgebildet ist und in dem zum Ergreifen des Gabelkopfs (12) mittels eines Handhabungsinstruments (70) jeweils wenigstens eine Haltenut (40) ausgebildet ist, die sich in einer Umfangsrichtung (42) des Gabelkopfs (12) und in einer Ebene orthogonal zur Längsrichtung (20) des Gabelkopfs (12) erstreckt, wobei die Haltenut (40) in einer die Längsrichtung (20) einschließenden gedachten Schnittebene betrachtet ausgehend von einer Oberfläche (38) des Umfangsbereichs (36) wenigstens abschnittsweise begrenzt ist
- von einem ersten nach radial innen erstreckten und in einer Ebene (54) orthogonal zur Längsrichtung (20) des Gabelkopfs (12) verlaufenden Flankenabschnitt (44),
- daran anschließend von einem zweiten in proximaler Richtung (28) erstreckten und konzentrisch zur Längsrichtung (20) verlaufenden Flankenabschnitt (46),
- daran anschließend von einem dritten nach radial innen erstreckten und in einer Ebene (56) orthogonal zur Längsrichtung (20) des Gabelkopfs (12) verlaufenden Flankenabschnitt (48),
- daran anschließend von einem vierten in distaler Richtung (30) erstreckten und konzentrisch zur Längsrichtung (20) verlaufenden Flankenabschnitt (50), der sich in distaler Richtung (30) bis unter den ersten Flankenabschnitt (44) erstreckt, und
- daran anschließend von einem fünften mit einer Komponente nach radial außen erstreckten und in einem Winkel zu einer Ebene (58) orthogonal zur Längsrichtung (20) des Gabelkopfs (12)distal geneigt verlaufenden Flankenabschnitt (52), wobei das Handhabungsinstrument (70) beidseits jeweils wenigstens einen nach radial innen erstreckten Vorsprung (78) aufweist und der jeweilige Vorsprung (78) einen in proximaler Richtung (28) erstreckten Ansatz (80) aufweist zur Ausbildung eines radialen Hintergriffs mit der Haltenut (40), um ein radiales Lösen des Handhabungsinstruments (70) aus dem Hintergriff in der Haltenut (40) zu verhindern und wobei der nach radial innen erstreckte Vorsprung (78) des Handhabungsinstruments (70) in einer die Längsrichtung (20) einschließenden gedachten Schnittebene betrachtet ausgehend von einer radial inneren Oberfläche (82) des Handhabungsinstruments (70) abschnittsweise begrenzt ist durch
- einen ersten nach radial innen erstreckten und in einer Ebene (83) orthogonal zur Längsrichtung (20) des Gabelkopfs (12) verlaufenden Flächenabschnitt (84),
- einen zweiten in proximaler Richtung (28) erstreckten und konzentrisch zur Längsrichtung (20) verlaufenden Flächenabschnitt (86), der von radial innen an den zweiten Flankenabschnitt (46) des Gabelkopfs (12) flächenhaft anlegbar ist,
- einen dritten nach radial innen gerade oder verrundet verlaufenden Flächenabschnitt (88),
- einen vierten in distaler Richtung (30) erstreckten und konzentrisch zur Längsrichtung (20) verlaufenden Flächenabschnitt (90), der sich in distaler Richtung (30) bis unter den ersten Flächenabschnitt (84) erstreckt,
- einen fünften mit einer Komponente nach radial außen erstreckten und in einem Winkel distal geneigt zu einer Ebene (93) orthogonal zur Längsrichtung (20) des Gabelkopfs (12) verlaufenden Flächenabschnitt (92), und wobeider fünfte Flächenabschnitt (92) des Vorsprungs (78) des Handhabungsinstruments (70) parallel zu dem fünften Flankenabschnitt (52) des Gabelkopfs (12) verläuft und wobei die radiale Erstreckung des ersten Flächenabschnitts (84) des Vorsprungs (78) des Handhabungsinstruments (70) wenigstens 20 %, insbesondere 20 % bis 100 % länger ist als diejenige des ersten Flankenabschnitts (40) des Gabelkopfs (12).

2. Sachgesamtheit nach Anspruch 1, **dadurch gekennzeichnet, dass** der fünfte Flankenabschnitt (52) ganz oder teilweise linear erstreckt verläuft.

3. Sachgesamtheit nach Anspruch 2, **dadurch gekennzeichnet, dass** der fünfte Flankenabschnitt (52) in einem Winkel zwischen 10 Grad und 50 Grad, insbesondere zwischen 20 Grad und 50 Grad, insbesondere zwischen 30 Grad und 50 Grad, insbesondere zwischen 30 und 40 Grad zu einer Ebene (58) orthogonal zur Längsrichtung (20) verläuft.

4. Sachgesamtheit nach Anspruch 1, 2 oder 3, **dadurch gekennzeichnet, dass** der fünfte Flankenabschnitt (52) radial außen bogenförmig gekrümmt ausläuft und in die Oberfläche (38) des äußeren Umfangsbereichs (36), vorzugsweise stetig, übergeht.

5. Sachgesamtheit nach einem oder mehreren der vorstehenden Ansprüche, **dadurch gekennzeichnet, dass** sich der vierte Flankenabschnitt (50) wenigstens um das Doppelte einer Wandstärke des Gabelkopfs (12) zwischen der Oberfläche (38) des Umfangsbereichs (36) und dem zweiten Flankenabschnitt (46) in distaler Richtung (30) erstreckt.

6. Sachgesamtheit nach einem oder mehreren der vorstehenden Ansprüche, **dadurch gekennzeichnet, dass** zwischen dem ersten und dem zweiten Flächenabschnitt (84, 86) des Vorsprungs (78) des Handhabungsinstruments (70) ein weiterer bogenförmig gekrümmt oder linear verlaufender Flächenabschnitt (94) ausgebildet ist.

7. Sachgesamtheit nach Anspruch 6, **dadurch gekennzeichnet, dass** der weitere Flächenabschnitt (94) in einem Winkel zwischen 20 Grad und 40 Grad zur Ebene (83)orthogonal zur Längsrichtung (20) verläuft.

8. Sachgesamtheit nach einem oder mehreren der vorstehenden Ansprüche, **dadurch gekennzeichnet, dass** die Längsabmessung des zweiten Flächenabschnitts (86) des Vorsprungs (78) des Handhabungsinstruments (70) kürzer ist als diejenige des zweiten Flankenabschnitts (46) des Gabelkopfs (12).

## Claims

1. Assembly of an osteosynthesis device (2) and a handling instrument (70) for gripping a fork head (12) of the osteosynthesis device (2) by engaging on both sides in a respective holding groove (40) of the fork head (12);
wherein the osteosynthesis device (2) is configured with a bone anchor (4), in particular a bone screw (6), with a shaft (8) and a head (14), and with a fork head (12) which is U-shaped in a side view and has a receiving opening (34) for a correction element, in particular a correction rod, and has two legs (32); wherein the head (14) of the bone anchor (4) and the fork head (12) are either rigidly attached to each other, in particular integrally, or the head (14) of the bone anchor (4) is mounted pivotably on a distal end (26) of the fork head (12), and in this case the fork head (12) can be fixed in a pivot position intended by the surgeon relative to the head (14) of the bone anchor (4) which is or can be secured in the bone, wherein the fork head (12) has a longitudinal direction (20) and a direction (22) which is radial thereto, and a distal end (26) adjacent to the bone anchor (4) and a proximal end (24) facing away from this in the longitudinal direction (20), so that also a distal direction (30) and a proximal direction (28) are defined; wherein the legs (32) extend in the proximal direction (28) starting from the distal region of the fork head (12) and between them delimit the receiving opening (34) for the correction element; wherein the legs (32) have a radially outer peripheral region (36) in the form of an arcuate curve, in particular in the form of a circle arc, and in which at least one respective holding groove (40) is formed for gripping of the fork head (12) by means of a handling instrument (70), which groove extends in a peripheral direction (42) of the fork head (12) and in a plane orthogonal to the longitudinal direction (20) of the fork head (12); wherein the holding groove (40), viewed in a theoretical sectional plane enclosing the longitudinal direction (20), starting from a surface (38) of the peripheral region (36), is delimited at least in portions by:
- a first flank portion (44) which extends radially inwardly and runs in a plane (54) orthogonal to the longitudinal direction (20) of the fork head (12),
- adjoining this, a second flank portion (46) which extends in the proximal direction (28) and runs concentrically to the longitudinal direction (20),
- adjoining this, a third flank portion (48) which extends radially inwardly and runs in a plane (56) orthogonal to the longitudinal direction (20) of the fork head (12),
- adjoining this, a fourth flank portion (50) which extends in the distal direction (30) and runs concentrically to the longitudinal direction (20), which flank portion extends in the distal direction (30) to under the first flank portion (44), and
- adjoining this, a fifth flank portion (52) which has a radially outwardly extending component and runs sloping distally at an angle to a plane (58) orthogonal to the longitudinal direction (20) of the fork head (12); wherein the handling instrument (70) has at least one radially inwardly extending protrusion (78) on both sides, and the respective protrusion (78) has an attachment (80) extending in the proximal direction (28) to form a radial undercut with the holding groove (40) in order to prevent a radial release of the handling instrument (70) from the undercut in the holding groove (40);
and wherein the radially inwardly extending protrusion (78) of the handling instrument (70), viewed in a theoretical sectional plane enclosing the longitudinal direction (20), starting from a radially inner surface (82) of the handling instrument (70), is delimited in portions by:
- a first surface portion (84) which extends radially inwardly and runs in a plane (83) orthogonal to the longitudinal direction (20) of the fork head (12),
- a second surface portion (86) which extends in the proximal direction (28) and runs concentrically to the longitudinal direction (20), which portion, radially from the inside, can be laid superficially against the second flank portion (46) of the fork head (12),
- a third surface portion (88) which runs radially inwardly in straight or rounded fashion,
- a fourth surface portion (90) which extends in the distal direction (30) and runs concentrically to the longitudinal direction (20), which surface portion extends in the distal direction (30) to under the first surface portion (84),
- a fifth surface portion (92) which has a radially outwardly extending component and runs sloping distally at an angle to a plane (93) orthogonal to the longitudinal direction (20) of the fork head (12); and wherein the fifth surface portion (92) of the protrusion (78) of the handling instrument (70) runs parallel to the fifth flank portion (52) of the fork head (12); and wherein the radial extension of the first surface portion (84) of the protrusion (78) of the handling instrument (70) is at least 20%, in particular 20% to 100% longer than that of the first flank portion (40) of the fork head (12).

2. Assembly according to claim 1, **characterised in that** the fifth flank portion (52) has a fully or partially linear extent.

3. Assembly according to claim 2, **characterised in that** the fifth flank portion (52) runs at an angle of between 10° and 50°, in particular between 20° and 50°, in particular between 30° and 50°, in particular between 30° and 40°, to a plane (58) orthogonal to the longitudinal direction (20) .

4. Assembly according to claim 1, 2 or 3, **characterised in that** the fifth flank portion (52) runs out radially outwardly in the form of an arcuate curve and transforms preferably constantly into the surface (38) of the outer peripheral region (36).

5. Assembly according to one or more of the preceding claims, **characterised in that** the fourth flank portion (50) extends in the distal direction between the surface (38) of the peripheral region (36) and the second flank portion (46) by an extent equal to at least twice a wall thickness of the fork head (12).

6. Assembly according to one or more of the preceding claims, **characterised in that** a further surface portion (94) in the form of an arcuate curve or running linearly is formed between the first and second surface portions (84, 86) of the protrusion (78) of the handling instrument (70).

7. Assembly according to claim 6, **characterised in that** the further surface portion (94) runs at an angle of between 20° and 40° to the plane (83) orthogonal to the longitudinal direction (20).

8. Assembly according to one or more of the preceding claims, **characterised in that** the linear dimension of the second surface portion (86) of the protrusion (78) of the handling instrument (70) is shorter than that of the second flank portion (46) of the fork head (12).

## Revendications

1. Ensemble comprenant un dispositif d'ostéosynthèse (2) et un instrument de manipulation (70) pour saisir une chape (12) du dispositif d'ostéosynthèse (2) par un engagement de part et d'autre dans une rainure de retenue (40) respective de la chape (12),
dans lequel ledit dispositif d'ostéosynthèse (2) est réalisé avec un ancre à os (4) présentant une tige (8) et une tête (14), en particulier avec une vis à os (6), et avec une chape (12) ayant deux branches (32) qui est en forme d'U dans une vue de côté et présente une ouverture de logement (34) pour un élément de correction, en particulier une tige correctrice, dans lequel ladite tête (14) de l'ancre à os (4) et la chape (12) soit sont réalisées de manière rigide l'une avec l'autre, en particulier d'un seul tenant, soit la tête (14) de l'ancre à os (4) est logée à pivotement à une extrémité distale (26) de la chape (12) et, dans ce cas, la chape (12) peut être fixée dans une position de pivotement visée par le chirurgien, par rapport à la tête (14) de l'ancre à os (4) immobilisé ou apte à être immobilisé dans l'os, ladite chape (12) présentant une direction longitudinale (20) et une direction (22) radiale par rapport à celle-ci ainsi qu'une extrémité distale (26) adjacente à l'ancre à os (4) et une extrémité proximale (24) opposée à celle-ci dans la direction longitudinale (20) de sorte qu'une direction distale (30) et une direction proximale (28) sont définies aussi, dans lequel les branches (32) s'étendent dans la direction proximale (28) à partir d'une zone distale de la chape (12) et délimitent entre elles l'ouverture de logement (34) pour l'élément de correction, dans lequel les branches (32) présentent une zone périphérique radialement extérieure (36) qui est courbée en arc, en particulier en arc de cercle, et dans laquelle, pour saisir la chape (12) au moyen d'un instrument de manipulation (70), est ménagé respectivement au moins une rainure de retenue (40) qui s'étend dans une direction circonférentielle (42) de la chape (12) et dans un plan orthogonal à la direction longitudinale (20) de la chape (12), dans lequel la rainure de retenue (40), vue dans un plan de coupe imaginaire incluant la direction longitudinale (20), est délimitée au moins par sections, à partir d'une surface (38) de la zone périphérique (36),
- par une première section de flanc (44) qui s'étend radialement vers l'intérieur et dans un plan (54) orthogonal à la direction longitudinale (20) de la chape (12),
- par une deuxième section de flanc (46) contiguë à cette dernière, qui s'étend dans la direction proximale (28) et de façon concentrique à la direction longitudinale (20),
- par une troisième section de flanc (48) contiguë à cette dernière, qui s'étend radialement vers l'intérieur et dans un plan (56) orthogonal à la direction longitudinale (20) de la chape (12),
- par une quatrième section de flanc (50) contiguë à cette dernière, qui s'étend dans la direction distale (30) et de manière concentrique par rapport à la direction longitudinale (20) et qui s'étend dans la direction distale (30) jusqu'au-dessous de la première section de flanc (44), et
- par une cinquième section de flanc (52) contiguë à cette dernière, qui s'étend radialement vers l'extérieur avec un composant et s'étend à inclinaison distale à un angle par rapport à un plan (58) orthogonal à la direction longitudinale (20) de la chape (12), dans lequel l'instrument de manipulation (70) présente de part et d'autre respectivement au moins une saillie (78) s'étendant radialement vers l'intérieur, et la saillie (78) respective présente un épaulement (80) s'étendant dans la direction proximale (28) pour réaliser un engagement de derrière radial avec la rainure de retenue (40) afin d'empêcher que l'instrument de manipulation (70) ne soit dégagé radialement de l'engagement de derrière dans la rainure de retenue (40), et
dans lequel, vue dans un plan de coupe imaginaire incluant la direction longitudinale (20), la saillie (78) s'étendant radialement vers l'intérieur de l'instrument de manipulation (70) est délimitée par sections, à partir d'une surface radialement intérieure (82) de l'instrument de manipulation (70), par
- une première section de surface (84) qui s'étend radialement vers l'intérieur et dans un plan (83) orthogonal à la direction longitudinale (20) de la chape (12),
- une deuxième section de surface (86) qui s'étend dans la direction proximale (28) et de façon concentrique par rapport à la direction longitudinale (20) et qui peut être appliquée en plan radialement de l'intérieur à la deuxième section de flanc (46) de la chape (12),
- une troisième section de surface (88) qui s'étend de manière droite ou arrondie radialement vers l'intérieur,
- une quatrième section de surface (90) qui s'étend dans la direction distale (30) et de manière concentrique par rapport à la direction longitudinale (20) et qui s'étend dans la direction distale (30) jusqu'au-dessous de la première section de surface (44),
- une cinquième section de surface (92) qui s'étend radialement vers l'extérieur avec un composant et s'étend à inclinaison distale à un angle par rapport à un plan (93) orthogonal à la direction longitudinale (20) de la chape (12), et dans lequel la cinquième section de surface (92) de la saillie (78) de l'instrument de manipulation (70) s'étend parallèlement à la cinquième section de flanc (52) de la chape (12), et dans lequel l'extension radiale de la première section de surface (84) de la saillie (78) de l'instrument de manipulation (70) est d'au moins 20 %, en particulier de 20 % à 100 % plus longue que celle de la première section de flanc (40) de la chape (12).

2. Ensemble selon la revendication 1, **caractérisé par le fait que** la cinquième section de flanc (52) s'étend complètement ou en partie de manière linéaire.

3. Ensemble selon la revendication 2, **caractérisé par le fait que** la cinquième section de flanc (52) s'étend à un angle compris entre 10 degrés et 50 degrés, en particulier entre 20 degrés et 50 degrés, en particulier entre 30 degrés et 50 degrés, en particulier entre 30 et 40 degrés, par rapport à un plan (58) orthogonal à la direction longitudinale (20).

4. Ensemble selon la revendication 1, 2 ou 3, **caractérisé par le fait que** la cinquième section de flanc (52) se termine radialement à l'extérieur en étant courbée en arc et débouche, de préférence de façon continue, dans la surface (38) de la zone périphérique extérieure (36).

5. Ensemble selon l'une ou plusieurs des revendications précédentes, **caractérisé par le fait que** la quatrième section de flanc (50) s'étend au moins du double d'une épaisseur de paroi de la chape (12) entre la surface (38) de la zone périphérique (36) et la deuxième section de flanc (46) dans la direction distale (30).

6. Ensemble selon l'une ou plusieurs des revendications précédentes, **caractérisé par le fait qu'**une autre section de surface (94) s'étendant de manière courbée en arc ou linéaire est formée entre les première et deuxième sections de surface (84, 86) de la saillie (78) de l'instrument de manipulation (70).

7. Ensemble selon la revendication 6, **caractérisé par le fait que** ladite autre section de surface (94) s'étend à un angle compris entre 20 degrés et 40 degrés par rapport au plan (83) orthogonal à la direction longitudinale (20).

8. Ensemble selon l'une ou plusieurs des revendications précédentes, **caractérisé par le fait que** la dimension longitudinale de la deuxième section de surface (86) de la saillie (78) de l'instrument de manipulation (70) est plus courte que celle de la deuxième section de flanc (46) de la chape (12).
